# EUROPÄISCHE PATENTANMELDUNG

(11) **EP 2 727 900 A1**
(43) Veröffentlichungstag der Anmeldung: **07.05.2014**
(21) Anmeldenummer: 12191092.1
(22) Anmeldetag: 02.11.2012
(51) Int. Cl.: C07C 29/80, C10L 1/02

(54) **Verfahren zur Verarbeitung von Rohglyzerin und Energiegewinnung**

(71) Anmelder: ANTAN Investment Eins GmbH & Co. KG, 60323 Frankfurt am Main (DE)
(72) Erfinder: Mehrling, Peter, 64293 Darmstadt (DE)
(74) Vertreter: WSL Patentanwälte Partnerschaft mbB

(57) **Zusammenfassung**

Um ein Verfahren bereitzustellen, mit dem Abfallprodukte aus der Verwertung biogener Rohstoffquellen, insbesondere der Herstellung von Biodiesel, einfach und mit geringem Energiebedarf einer weiteren Verwendung zugeführt werden können wird erfindungsgemäß ein Verfahren zur Verarbeitung von Rohglyzerin vorgeschlagen, das die folgenden Stufen umfasst:
a) Erzeugung eines ersten Zwischenprodukts durch Erhitzen des Rohglyzerins auf eine Temperatur, die dazu geeignet ist, die im Rohglyzerin enthaltenen Stoffe abzudestillieren, die bei Normaldruck eine Siedetemperatur im Bereich von 60 °C bis 80 °C aufweisen,
b) Erzeugung eines zweiten Zwischenprodukts durch Erhitzen des Zwischenprodukts aus Stufe a) auf eine Temperatur, die dazu geeignet ist, die im Rohglyzerin enthaltenen Stoffe abzudestillieren, die bei Normaldruck eine Siedetemperatur im Bereich von 80 °C bis 100 °C aufweisen, und
c) Filtrieren des Zwischenprodukts aus Stufe b) zur Abtrennung von darin enthaltenen Feststoffen, unter Erhalt eines aufbereiteten Glyzerins.

## Beschreibung

Die vorliegende Erfindung betrifft ein Verfahren zur Verarbeitung von Rohglyzerin und Energiegewinnung.

### 1. Technischer Hintergrund der Erfindung

Der Verwendung von Produkten aus biogenen Quellen, d.h. nachwachsenden Rohstoffquellen wie Pflanzen, kommt insbesondere in Anbetracht der schwindenden Öl- und Gasvorkommen eine immer größere Bedeutung zu. Es werden Fette aus Pflanzenölen wie Rapsöl, Palmöl und Jatropha-Öl, Harze aus Nadelhölzern und Koniferen, Zellulose, Hemizellulose und Lignin aus Bambus, Getreidestroh, Reisstroh und Fruchtschalen gewonnen, um nur einige Beispiele für die Gewinnung von Stoffen aus biogenen Quellen zu nennen.

Ein wichtiges Einsatzgebiet nachwachsender Rohstoffquellen ist auch die Gewinnung von Biotreibstoffen, bei denen zum Beispiel durch Umesterung eine Erzeugung von Methylestern erfolgt. Bei diesem Verfahrensschritt fallen unter anderem große Mengen von Glyzerin an, die aufgrund zahlreicher Verunreinigungen durch anorganische und organische Substanzen sowie Wasser als Rohglyzerin bezeichnet werden.

Es sind verschiedene Verfahren bekannt, bei denen Rohglyzerin unter großem verfahrentechnischem Aufwand und/oder hohem Energiebedarf aufgereinigt und z.B. zur weiteren Verwendung in der chemischen und pharmazeutischen Industrie in reiner Form dargestellt werden.

### 2. Technische Aufgabe der Erfindung

Ausgehend davon lag der vorliegenden Erfindung die Aufgabe zugrunde, ein Verfahren bereitzustellen, mit dem Abfallprodukte aus der Verwertung biogener Rohstoffquellen, insbesondere der Herstellung von Biodiesel, einfach und mit geringem Energiebedarf einer weiteren Verwendung zugeführt werden können.

### 3. Merkmale der Erfindung

Die oben angegebene Aufgabe wird erfindungsgemäß gelöst durch ein Verfahren zur Verarbeitung von Rohglyzerin, das die folgenden Stufen umfasst:
a) Erzeugung eines ersten Zwischenprodukts durch Erhitzen des Rohglyzerins auf eine Temperatur, die dazu geeignet ist, die im Rohglyzerin enthaltenen Stoffe abzudestillieren, die bei Normaldruck eine Siedetemperatur im Bereich von 60 °C bis 80 °C aufweisen,
b) Erzeugung eines zweiten Zwischenprodukts durch Erhitzen des Zwischenprodukts aus Stufe a) auf eine Temperatur, die dazu geeignet ist, die im Rohglyzerin enthaltenen Stoffe abzudestillieren, die bei Normaldruck eine Siedetemperatur im Bereich von 80 °C bis 100 °C aufweisen, und
c) Filtrieren des Zwischenprodukts aus Stufe b) zur Abtrennung von darin enthaltenen Feststoffen, unter Erhalt eines aufbereiteten Glyzerins.

Bei dem erfindungsgemäßen Verfahren erfolgt die Abtrennung der niedrig siedenden Substanzen und des Wasseranteils demnach in verschiedenen aufeinanderfolgenden Stufen. Durch das erfindungsgemäße Verfahren ist daher eine schnelle und kostengünstige Aufbereitung von Rohglyzerin möglich, bei der eine starke Absenkung des Wassergehalts sowie insbesondere die Abtrennung von anorganischen Substanzen wie Kalium, Kalzium und Natriumsalzen von Carbonsäuren erfolgt.

Unter dem Begriff Rohglyzerin im Sinne der Erfindung wird ein flüssiges Gemisch mit einem Glyzerinanteil von wenigstens 67 Gew.-% verstanden, das zusätzlich auch einen Mineralstoffanteil, d.h. einen Anteil an Ionen wie Natrium, Kalium, Calcium, Magnesium sowie Chlor und Phosphor von 2 Gew.-% bis 8 Gew.-%, einen Wasseranteil von 5 bis 20 Gew.-% und einen Anteil an organischen Verunreinigungen, wie z.B. Ethanol und Methanol, von 0,2 bis 5 Gew.-% aufweist.

Unter "destillieren" oder "abdestillieren" wird im Sinne der vorliegenden Erfindung verstanden, dass ein flüssiges Ausgangsgemisch zum Sieden gebracht wird, um die unter den gewählten Destillationbedingungen flüchtigen Komponenten des Ausgangsgemisches in die Gasphase zu überführen.

In der Stufe a) werden die im Rohglyzerin enthaltenen Stoffe abdestilliert, die bei Normaldruck eine Siedetemperatur im Bereich von 60 °C bis 80 °C aufweisen. Hierfür wird das Rohglyzerin in der Stufe a) entweder bei Normaldruck auf eine Temperatur in dem Bereich von 85 °C bis 95 °C erhitzt oder bei verringertem Druck auf eine entsprechend verringerte Temperatur, bei der die im Rohglyzerin enthaltenen Stoffe abdestilliert werden, die bei Normaldruck eine Siedetemperatur im Bereich von 60 °C bis 80 °C aufweisen. In einer Ausführungsform wird daher das Rohglyzerin nicht wie bei einem Normaldruck von 1013,25 hPa (mbar) auf eine Temperatur in dem Bereich von 85 °C bis 95 °C erhitzt sondern unter einem verringertem Druck von 500 hPa (mbar) auf eine Temperatur im Bereich von 30 °C bis 50 °C.

In einer alternativen Ausführungsform erfolgt in der Stufe a) zunächst in einer ersten Phase a1) das Erhitzen des Rohglyzerins auf die oben genannte Temperatur unter Normaldruck und anschließend in einer zweiten Phase a2) auf die oben genannte Temperatur unter verringertem Druck. Auf diese Weise werden die im Rohglyzerin enthaltenen Stoffe, die bei Normaldruck eine Siedetemperatur im Bereich von 60 °C bis 80 °C aufweisen, besonders effizient entfernt.

In der Stufe a) werden entweder bei Normaldruck oder bei einem verringertem Druck oder zunächst bei Normaldruck und anschließend bei verringertem Druck insbesondere die im Rohglyzerin enthaltenen Anteile an Methanol und Ethanol abdestilliert. In einer Ausführungsform werden diese Stoffe abgesaugt.

In einer Ausführungsform wird vor Stufe a) eine Vorfiltration durchgeführt. Bei dieser Vorfiltration werden grobe partikuläre Verunreinigungen abgetrennt, die insbesondere bei Lagerung und beim Transport unbeabsichtigt eingebracht worden sein können.

In der Stufe b) werden aus dem Zwischenprodukt, das in der Stufe a) erzeugt wurde, die im Rohglyzerin enthaltenen Stoffe abdestilliert, die bei Normaldruck eine Siedetemperatur im Bereich von 80 °C bis 100 °C aufweisen. Hierfür wird das Zwischenprodukt, das in der Stufe a) erzeugt wurde, in der Stufe b) entweder bei Normaldruck auf eine Temperatur in dem Bereich von 110 °C bis 140 °C erhitzt oder bei verringertem Druck auf eine entsprechend verringerte Temperatur, bei der die im Rohglyzerin enthaltenen Stoffe abdestilliert werden, die bei Normaldruck eine Siedetemperatur im Bereich von 80 °C bis 100 °C aufweisen. In einer Ausführungsform wird daher das Zwischenprodukt, das in der Stufe a) erzeugt wurde, nicht wie bei einem Normaldruck von 1013,25 hPa (mbar) auf eine Temperatur in dem Bereich von 110 °C bis 140 °C erhitzt sondern unter einem verringertem Druck von 500 hPa (mbar) auf eine Temperatur im Bereich von 50 °C bis 70 °C.

In einer alternativen Ausführungsform erfolgt in der Stufe b) zunächst in einer ersten Phase b1) das Erhitzen des Zwischenprodukts, das in der Stufe a) erzeugt wurde, auf die oben genannte Temperatur unter Normaldruck und anschließend in einer zweiten Phase b2) auf die oben genannte Temperatur unter verringertem Druck. Auf diese Weise werden die im Rohglyzerin enthaltenen Stoffe, die bei Normaldruck eine Siedetemperatur im Bereich von 80 °C bis 100 °C aufweisen, besonders effizient entfernt.

In der Stufe b) werden entweder bei Normaldruck oder bei einem verringertem Druck oder zunächst bei Normaldruck und anschließend bei verringertem Druck insbesondere die im Rohglyzerin enthaltenen Anteile an Wasser abdestilliert bzw. stark reduziert. Durch das Abdestillieren des Wassers werden gleichzeitig zuvor durch das Wasser in Lösung gehaltene Salze von Carbonsäuren, insbesondere Natrium- und Kaliumsalze von Carbonsäuren, als Feststoffe gefällt.

Diese auskristallisierten und gefällten Feststoffe werden anschließend in Stufe c) durch Filtrieren abgetrennt. In Stufe c) wird in einer Ausführungsform das gesamte aus der Stufe b) erhaltene Zwischenprodukt einer Filtration unterworfen.

In einer Ausführungsform wird das aus der Stufe b) erhaltene Zwischenprodukt in der Prozessstufe c) in einen oberen, feststoffarmen Anteil und in einen unteren feststoffreichen Anteil getrennt. Anschließend werden der feststoffarme Anteil und der feststoffreiche Anteil separat voneinander einer Filtration unterzogen. Durch die separate Filtration des oberen und unteren Anteils, bei der der untere Anteil vorzugsweise mehreren Filtrationsstufen unterworfen werden kann, wird eine bessere Abtrennung der Feststoffe von dem Gemisch gewährleistet. Insbesondere ist diese Ausführung dann vorteilhaft, wenn im Zusammenhang mit den gewählten Filtrierverfahren der apparative Aufwand verringert wird, d.h. die Flächenleistung der Filter steigt oder der Bedarf an Zuschlagsstoffe in Summe sinkt.

Die Filtration kann mit unterschiedlichen Filtertypen erfolgen. In einer Ausführungsform erfolgt die Filtration unter Verwendung von Keramikfiltern und Filterkerzen. Diese Filter sind feinporig und haben vorzugsweise eine Porengröße von 0,1 bis 10 µm (Mikrometer).

In einer Ausführungsform werden in Stufe c) vor dem Filtrieren Filtrierhilfsstoffe zugegeben. Vorzugsweise wird als Filtrierhilfsstoff Zellulose verwendet. Die zugefügte Zellulosefasern bilden auf den Filtern zusammen mit den abgeschiedenen Substanzen ein Filterkuchen. In einer weiteren Ausführungsform wird dem Zwischenprodukt aus der Stufen b) vor der Stufe c) ein Fällungsmittel zugegeben, um die Ausbildung eines stabilen Filterkuchens zu bewirken.

Da die Feststoffe mit dem Glyzerin eine schmierige, pampige Masse bilden, wird die Filtration vorzugsweise als Druckfiltration ausgeführt, wobei der Mindestdruck hierbei 1 bar Überdruck (2 bar absolut = 2000 hPa absolut) betragen sollte. In einer bestimmten Ausführungsform erfolgt die Filtration in Stufe c) unter einem absoluten Druck in dem Bereich von 5500 hPa bis 6500 hPa (5,5 bis 6,5 bar absolut). Hierdurch wird mit vertretbarem technischen Aufwand die Filtriergeschwindigkeit erhöht.

Eine Filterreinigung erfolgt in einer bestimmten Ausführungsform durch Rückspülung. Das nach der Filtration verbleibende feststofffreie Filtrat ist das aufbereitete Glyzerin, das für die Weiterverarbeitung und insbesondere Verbrennung geeignet ist.

Eine Einstellung des pH-Werts des erhaltenen aufbereiteten Glyzerins erfolgt vorzugsweise auf einen neutralen pH-Bereich, d.h. einen Bereich von zwischen pH 6,5 und 7,5. In einer Ausführung wird diese pH-Einstellung durch Addition eines entsprechenden Puffers nach der Fil t-ration in Prozessstufe c) durchgeführt.

### 4. Vorteile der Erfindung

Bei dem erfindungsgemäßen Verfahren wird aus Rohglyzerin ein aufbereitetes Glyzerin erzeugt, das in zahlreichen weiteren Prozessen verwendbar ist. Insbesondere kann das Produkt der Erzeugung von mechanischer Energie durch dessen Einsatz in einem thermodynamischen Prozess mit innerer Verbrennung dienen. In einer bevorzugten Anwendung wird das erfindungsgemäß erzeugte aufbereitete Glyzerin in einem langsam laufenden Zweitaktmotor im Leistungsbereich von 1 bis 100 MW eingesetzt. Dabei ist der hohe thermodynamische Wirkungsgrad und die Toleranz dieser Motoren gegenüber den auch im aufbereiteten Rohglyzerin noch enthaltenden Verunreinigungen entscheidend.

Die Energieerzeugung auf Basis des aufbereiteten Glyzerins nach dem erfindungsgemäßen Verfahren mittels Prozessen mit innerer Verbrennung erfolgt CO₂-neutral, wenn die eingesetzten Ausgangsprodukte biogenen Ursprungs sind und dadurch die bei der Verbrennung freigesetzte CO₂-Menge zuvor beim Wachstum der Pflanzen assimiliert wurde.

Der Anteil der Mineralstoffe und des Wassers des aufbereiteten Glyzerins zeigt deutlich geringere Werte im Vergleich zum Rohglyzerin entsprechend der zuvor gegebenen Definition. Vorzugsweise weist das nach dem erfindungsgemäßen Verfahren erzeugte aufbereitete Glyzerin einen Mineralstoffanteil, d.h. einen Anteil an Ionen wie Natrium, Kalium, Calcium, Magnesium sowie Chlor und Phosphor, im Bereich von lediglich 0,05 Gew.-% bis 0,2 Gew.-% auf.Vorzugsweise liegt der Wasseranteil lediglich im Bereich von 0,5 bis 1,5 Gew.-%. Der Anteil an organischen Verunreinigungen, wie Ethanol, Methanol, liegt vorzugsweise im Bereich von 0,02 bis 0,5 Gew.-%.

Insgesamt weist das nach dem erfindungsgemäßen Verfahren erzeugte aufbereitete Glyzerin vorzugsweise eine Reinheit in dem Bereich von 97,5 bis 99,5 Gew.-% auf.

Der Heizwert des Rohglyzerins liegt im Bereich von 13 bis 15,5 MJ/kg. Das nach dem erfindungsgemäßen Verfahren erzeugte aufbereitete Glyzerin kann dagegen je nach Ausführungsform einen Heizwert von 15,5 MJ/kg bis 18,5 MJ/kg erreichen.

### 5. Modifikation des Heizwertes des aufbereiteten Glyzerins

Um den Heizwert des aufbereiteten Glyzerins zu modifizieren können diesem heizwertreiche biogene flüssige Substanzen zugegeben werden, die einen höheren Brennwert aufweisen als das nach dem oben beschriebenen Verfahren aufbereitete Glyzerin.

In einer Ausführungsform wird dem aufbereiteten Glyzerin zur Erhöhung des Heizwertes eine biogene Flüssigkeit zugesetzt, die ausgewählt sein kann unter Pflanzenöl, Biodiesel und Gemischen davon. Das Pflanzenöl ist vorzugsweise ausgewählt unter Rapsöl, Sojaöl, Sonnenblumenöl, Palmöl und Jatropha-Öl. Unter Biodiesel versteht man im Wesentlichen ein durch Umesterung von Rapsöl oder Sojaöl mit Methanol gewonnenes Gemisch aus Fettsäuremethylestern. Diese umfassen im Wesentlichen C16-C18-Fettsäuremethylester sowie ungesättigte C18-Fettsäuremethylester.

Durch Zugabe derartiger Stoffe bzw. Stoffgemischen mit höherem Brennwert kann der Brennwert des aufbereiteten Glyzerins auf Werte im Bereich von 20 000 bis 21 000 kJ/kg angehoben werden, die für eine effiziente Verbrennung in einem langsam laufenden Zweitakt-Dieselmotor notwendig sind. Nach der Zugabe der Substanzen mit höherem Brennwert wird das Gemisch vorzugsweise homogenisiert.

Um mögliche Verunreinigung, die durch die Zugabe der Substanzen mit höherem Brennwert zugefügt worden sein können, abzuscheiden, wird bei einer Ausführungsform das Gemisch zentrifugiert.

### 6. Mögliche Verwendung des aufbereiteten Rohglyzerins

Das aufbereitete Rohglyzerin kann zur Erzeugung mechanischer Energie mittels eines Prozesses mit innerer Verbrennung eingesetzt werden.

Die während der die Verbrennung des aufbereiteten Glyzerins entstehenden Abgase treten mit hoher Temperatur in dem Bereich von 250 bis 600°C aus dem Motor aus und können unmittelbar zur Dampferzeugung und damit ebenfalls zur Stromerzeugung eingesetzt werden. Vorzugsweise wird das Abgas zuvor durch eine Entstickungsanlage, in der die Stickoxidkonzentration reduziert wird, geleitet und vor Abgabe an die Umwelt über einen Staubfilter (z. B. Schlauch- oder Elektrofilter) filtriert.

In einer Ausführungsform wird ein langsam laufender Zweitakt-Dieselmotor in stationärer Ausführung mit direkt gekoppeltem Generator eingesetzt, so dass die erzeugte mechanische Energie direkt in elektrische Energie umgewandelt werden kann. In dieser Ausführungsform werden die Druckenergie des aus dem Verbrennungsprozess anfallenden Rauchgases zur Verdichtung der für selbigen Prozess benötigten Verbrennungsluft eingesetzt und die Enthalpie des mit 250 bis 350 °C anfallenden Auspuffgases (Rauchgas der Verbrennung) zur Dampferzeugung oder Prozesswärme Erzeugung eingesetzt, das Rauchgas wird dabei gleichzeitig abgekühlt.

Daneben fallen im Bereich der Aggregate-Kühlung und der Schmierölkühlung Prozesswärmeströme an, die als Prozesswärme exportiert werden kann. In jeder der Ausführungsformen dient dieser Enthalpie-Strom der Erhitzung des unbehandelten Rohglyzerins in den Prozessstufen a) und b). Dadurch kann die Effizienz des Gesamtverfahrens, das Aufbereitung von Rohglyzerin und Prozess mit innerer Verbrennung und Stromerzeugung umfasst, deutlich erhöht werden. Der Wirkungsgrad beträgt entsprechend dem Beispiel thermodynamisch 53 %.

Weitere Vorteile, Merkmale und Anwendungsmöglichkeiten der vorliegenden Erfindung werden anhand der folgenden Figurenbeschreibung und des weiter unten angegebenen Beispiels zur Durchführung einer speziellen Ausführungsform des Verfahrens deutlich.

### 7. Figurenbeschreibung

Bei den Figuren zeigen:
- Figur 1: eine schematische Darstellung eines ersten Teils einer möglichen Prozessanordnung zur Durchführung des erfindungsgemäßen Verfahrens
- Figur 2: eine schematische Darstellung des zweiten Teils einer möglichen Prozessanordnung zur Durchführung des erfindungsgemäßen Verfahrens.

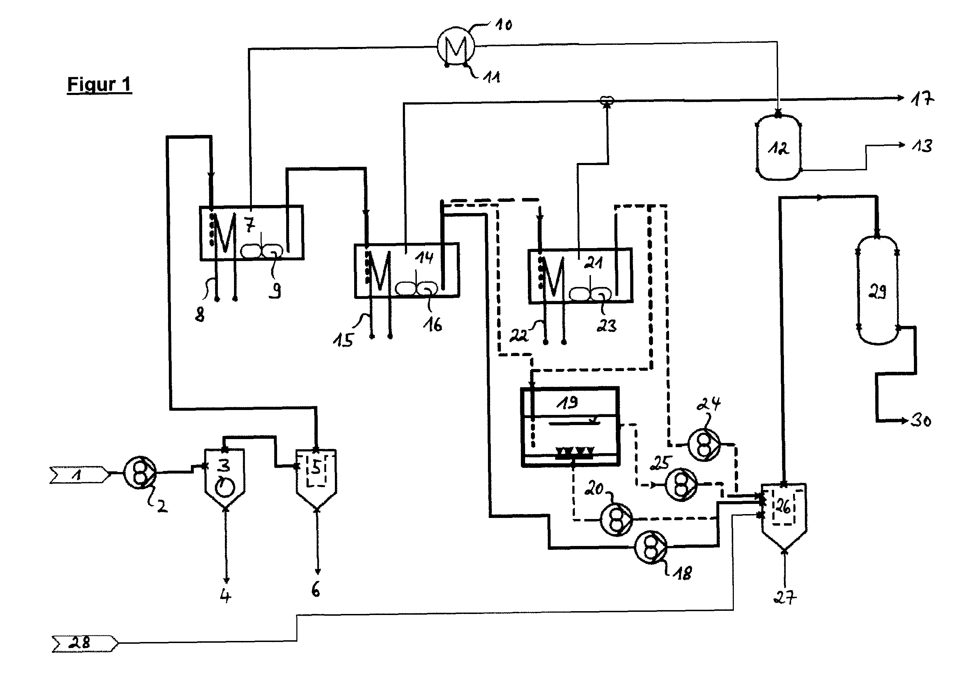

In Figur 1 wird der erste Teil einer möglichen Prozessanordnung zur Durchführung des erfindungsgemäßen Verfahrens dargestellt. Hierbei steht die Bezugsziffer 1 für den Stoffstrom des eingesetzten Rohglyzerins. Dieser Stoffstrom wird über die Rohglyzerinpumpe 2 zu einer Zen t-rifuge 3 zur Vorreinigung des Rohglyzerins transportiert. Die in der Zentrifuge abg etrennten Verunreinigungen sind mit der Bezugsziffer 4 gekennzeichnet.

Das entsprechend vorgereinigte Rohglyzerin wird von der Zentrifuge 3 dann zu einem Flüssigkeitsfilter 5 transportiert, in dem aus dem Rohglyzerin Feststoffe 6 abgetrennt werden.

Das entsprechend aufbereitete Rohglyzerin wird dann in einen Destillationsbehälter 7 überführt, indem das Rohglyzerin auf eine Temperatur erhitzt wird, die dazu geeignet ist, die im Rohglyzerin enthaltenen Stoffe abzudestillieren, die bei Normaldruck eine Siedetemperatur im Bereich von 60° C bis 80° C aufweisen (Stufe a) des erfindungsgemäßen Verfahrens). In dem Destillationsbehälter 7 ist ein Rührer 9 angeordnet. Das Erhitzen des Rohglyzerins in dem Destillationsbehälter 7 auf die nötige Temperatur erfolgt über die Wärmezufuhr 8.

Nach Abschluss der Stufe a) wird das aus dieser Stufe hervorgehende erste Zwischenprodukt aus dem Destillationsbehälter 7 in einen weiteren Destillationsbehälter 14 überführt. Auch in diesem Destillationsbehälter 14 ist ein Rührer 16 angeordnet. Außerdem ist in dem Destillationsbehälter 14 eine Wärmezufuhr 15 vorgesehen, die das aus der Stufe a) hervorgegangene Zwischenprodukt auf eine Temperatur erhitzt, die dazu geeignet ist, die im Rohglyzerin entha I-tenen Stoffe abzudestillieren, die bei Normaldruck eine Siedetemperatur im Bereich von 80° C bis 100° C aufweisen (Stufe b) des erfindungsgemäßen Verfahrens).

Das aus der Stufe b) hervorgehende zweite Zwischenprodukt wird je nach Reinheitsgrad weiteren Aufreinigungsprozessen unterworfen.

Ist der Reinheitsgrad bereits recht hoch, so kann das aufbereitete Rohglyzerin aus dem Destllationsbehälter 14 über die Pumpe 18 zum Filter 26 transportiert werden, über den im aufbereiteten Rohglyzerin noch enthaltene Feststoffe 27 abgetrennt werden können.

Unter Umständen kann es jedoch erforderlich sein, das aus der Stufe b) erhaltene zweite Zw i-schenprodukt vor dem Filtrieren auf dem Filter 26 einer weiteren Vorreinigungsstufe zu unte r-ziehen. Hierfür wird das entsprechend aufzubereitende zweite Zwischenprodukt aus dem Destillationsbehälter 14 in einen Absetzbehälter 19 überführt, in dem das aus dem Destillationsbehälter 14 erhaltene zweite Zwischenprodukt in einen feststoffarmen Teil und in einen feststofreichen Teil aufgetrennt wird. Der feststoffreiche Teil setzt sich in dem Absetzbehälter 19 unten ab und kann dann über die Pumpe 20 zum Filter 26 transportiert werden. Der feststoffarme Teil aus dem oberen Bereich des Absetzbehälters 19 wird über die Pumpe 25 zum Filter 26 transportiert.

In manchen Fällen ist es nötig, das aus der Stufe b) erhaltene zweite Zwischenprodukt einem weiteren Destillationsschritt zu unterwerfen. Hierfür wird das zweite Zwischenprodukt aus dem Destillationsbehälter 14 in einen weiteren Destillationsbehälter 21 überführt in dem ein Rührer 23 und die Wärmezufuhr 22 vorgesehen ist. In dem Destillationsbehälter 21 wird das aus Stufe b) erhaltene Zwischenprodukt erneut auf eine Temperatur erhitzt, bei der solche im Rohglyzerin enthaltenen Stoffe abdestilliert werden, die bei Normaldruck eine Siedetemperatur im Bereich von 80° C bis 100° C aufweisen.

Je nach Qualitätsgrad des bei der Destillation in Destillationsbehälter 21 enthaltenen Produkts wird dieses entweder über den Absetzbehälter 19 noch einmal weiter aufbereitet oder direkt über die Pumpe 24 zum Filter 26 geleitet.

Je nach Bedarf kann dem Filter 26 ein Filtrierhilfsmittel über den mit der Ziffer 28 gekennzeichneten Stoffstrom zugeführt werden.

Das filtrierte aufbereitete Rohglyzerin wird vom Filter 26 in den Zwischenbehälter 29 geleitet.

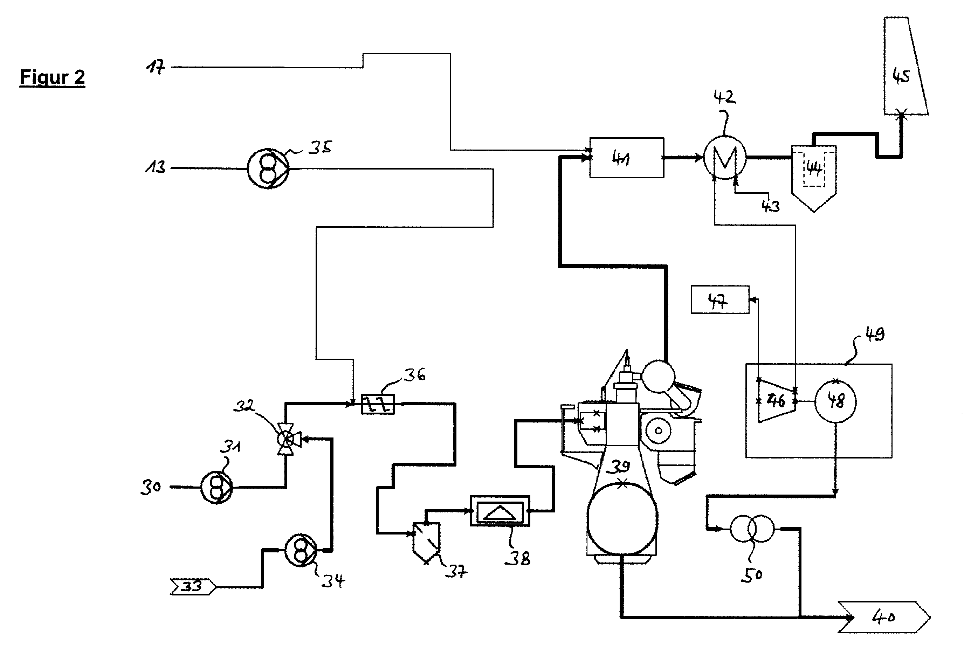

In Figur 2 wird der zweite Teil der in Figur 1 bereits teilweise dargestellten Prozessanordnung zur Durchführung des erfindungsgemäßen Verfahrens dargestellt. Über die Pumpe 31 wird das aufbereitete Glyzerin 30 zum Dreiwegemischventil 32 geleitet. Hier erfolgt die Zumischung des Stoffstromes 33, der über die Pumpe 34 transportiert wird. Bei dem Stoffstrom 33 handelt es sich um einen Stoff oder ein Stoffgemisch mit höherem Brennwert als das aufbereitete Glyzerin.

Das über das Mischventil 32 erzeugte Gemisch wird zu dem Homogenisator 36 geleitet. Kurz vor dem Homogenisator 36 werden dem Gemisch zusätzlich leicht siedende Komponenten 13, die über die Pumpe 35 gefördert werden, zugemischt. Diese leicht siedenden Komponenten 13 entstammen der Destillationsstufe a) und werden aus dem in Figur 1 dargestellten Destillationsbehälter 7 über den Kühler 10, der eine Kühlschleife 11 aufweist, und den Zwischenbehälter 12, die ebenfalls in Figur 1 dargestellt sind, und die Pumpe 35 transportiert.

Nach der Homogenisierung wird das aufbereitete und homogenisierte Glyzerin über den Filter 37 filtriert und anschließend in der Zentrifuge 38 zentrifugiert.

Das in der Zentrifuge 38 letztmalig aufbereitete Glyzerin wird dann in eine Anordnung 39 geleitet, die einen direkt mit einem Zweitaktmotor gekoppelten Generator umfasst. Die von dem Zweitaktmotor in der Anordnung 39 erzeugten Verbrennungsgase können in einer SCR-Anlage 41 entstickt werden. Über die Entstickungsanlage 41 wird auch in den in Figur 1 dargestellten Destillationsbehältern 14 und 21 verdampftes Wasser in das Verbrennungsgas abgeleitet.

Nach der Entstickungsanlage 41 ist ein Rauchgaskühler 42 angeordnet, der der Dampferzeugung aus Kesselspeisewasser 43 dient. Das gekühlte Rauchgas wird vom Rauchgaskühler 42 über den Rauchgasfilter 44 durch den Schornstein 45 abgeleitet.

Der im Rauchgaskühler 42 erzeugte Dampf wird zu einer Dampfturbine 46 geleitet, die mechanisch mit einem Generator 48 in einer Anordnung 49 gekoppelt ist. Der aus der Turbine 46 entströmende Niederdruckdampf gelangt zu dem Speisewassersystem 47, das die Dampfkondensation, Speisewassernachspeisung und die Versorgung des Rauchgaskühlers 42 mit Ke s-selspeisewasser 43 umfasst. Der im Generator 48 in der Anordnung 49 erzeugte elektrische Strom wird dann im Transformator 50 auf die Bedingungen des in der Anordnung 39 durch den direkt mit dem Zweitaktmotor gekoppelten Generator erzeugten elektrischen Stroms transformiert.

Mit der Bezugsziffer 40 ist die Summe des insgesamt durch den in der Anordnung 39 direkt mit dem Zweitaktmotor gekoppelten Generator und durch den in der Anordnung 49 vorgesehenen Generator 48 erzeugten elektrischen Stroms gekennzeichnet.

### 8. Ausführungsbeispiel

In diesem Beispiel wird die Energie- und Stoffbilanz für ein erfindungsgemäßes Verfahren dargestellt. Das erfindungsgemäße Verfahren wurde wie oben in der Beschreibung zu Figur 1 angegeben durchgeführt. Da es sich um ein kontinuierliches Verfahren handelt, wurden die mit einer Stunde Laufzeit erzielten Werte bestimmt. Die Bestimmung erfolgte rechnerisch, wobei die folgenden Werte ermittelt wurden: aus einer eingesetzten Menge von Rohglyzerin von 2375,4 kg wurden 2042,9 kg aufbereitetes Glyzerin erhalten. in dem Verfahren zur Aufbereitung des Glyzerins wurden 4,8 kg eines Alkohol-Azeotrops erhalten, das im Wesentlichen aus einem Methanol- Azeotrop besteht. Die Abtrennung dieses Azeotrops erfolgte in Stufe a). in Stufe b) wurden 190 kg Wasser abgetrennt. Durch die anschließende Filtration erfolgte eine Abtrennung von 95 kg festen Rückständen.

Das ursprünglich eingesetzte Rohglyzerin wies einen Heizwert von 15,5 MJ/kg auf. Mit dieser eingesetzten Menge wurden insgesamt elektrischer Strom mit einer Leistung 5,3 MW erzeugt, dabei liegt die elektrische Leistung der Stromerzeugung 1 bei 5,05 MW, der Stromerzeugung 2 bei 0,3 MW Neben der als elektrischer Strom entnommenen Energie wird zusätzlich Prozesswärme erzeugt. Die errechnete Leistung für Wärmeströme mit einer Temperatur von über 70 ·c beträgt 3 MW.

Aus den angegebenen Daten ergibt sich ein theoretisch errechneter Wirkungsgrad von 53,1 %, bezogen auf die erzeugte elektrische Energie, d.h. die Wärmeleistung wurde bei der Berechnung des thermodynamischen Wirkungsgrad nicht berücksichtigt.

Für Zwecke der ursprünglichen Offenbarung wird darauf hingewiesen, dass sämtliche Merkmale, wie sie sich aus der vorliegenden Beschreibung, der Zeichnung, dem Beispiel und den abhängigen Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen und die Betonung der Unabhängigkeit der einzelnen Merkmale voneinander wird hier nur der Kürze und der Lesbarkeit der Beschreibung wegen verzichtet.

Während die Erfindung im Detail in der Zeichnung, dem Beispiel und der vorangehenden Beschreibung dargestellt ist und beschrieben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten speziellen Ausführungsformen beschränkt.

### Bezugszeichenliste:

- 1: Rohglyzerin
- 2: Rohglyzerinpumpe
- 3: Zentrifuge
- 4: abgetrennte Verunreinigungen
- 5: Filter
- 6: abgetrennte Feststoffe
- 7: Destillationsbehälter
- 8: Wärmezufuhr
- 9: Rührer
- 10: Kühler
- 11: Kühlschleife
- 12: Zwischenbehälter
- 13: flüssige Leichtsieder
- 14: Destillationsbehälter
- 15: Wärmezufuhr
- 16: Rührer
- 17: verdampftes Wasser
- 18: Pumpe
- 19: Absetzbehälter
- 20: Pumpe
- 21: Destillationsbehälter
- 22: Wärmezufuhr
- 23: Rührer
- 24: Pumpe
- 25: Pumpe
- 26: Filter
- 27: abgetrennte Feststoffe
- 28: Filtrierhilfsmittel
- 29: Zwischenbehälter
- 30: aufbereitetes Glyzerin
- 31: Pumpe
- 32: Dreiwege-Mischventil
- 33: Stoff zur Erhöhung des Heizwertes
- 34: Pumpe
- 35: Pumpe
- 36: Homogenisator
- 37: Filter
- 38: Zentrifuge
- 39: Anordnung Zweitaktmotor mit Generator
- 40: elektrischer Strom
- 41: SCR-Anlage
- 42: Rauchgaskühler
- 43: Kesselspeisewasser
- 44: Rauchgasfilter
- 45: Schornstein
- 46: Dampfturbine
- 47: Speisewassersystem
- 48: Generator
- 49: Anordnung Dampfturbine/Generator
- 50: Transformator

## Patentansprüche

1. Verfahren zur Verarbeitung von Rohglyzerin, das die folgenden Stufen umfasst:
a) Erzeugung eines ersten Zwischenprodukts durch Erhitzen des Rohglyzerins auf eine Temperatur, die dazu geeignet ist, die im Rohglyzerin enthaltenen Stoffe abzudestillieren, die bei Normaldruck eine Siedetemperatur im Bereich von 60 °C bis 80 °C aufweisen,
b) Erzeugung eines zweiten Zwischenprodukts durch Erhitzen des Zwischenprodukts aus Stufe a) auf eine Temperatur, die dazu geeignet ist, die im Rohglyzerin enthaltenen Stoffe abzudestillieren, die bei Normaldruck eine Siedetemperatur im Bereich von 80 °C bis 100 °C aufweisen, und
c) Filtrieren des Zwischenprodukts aus Stufe b) zur Abtrennung von darin enthaltenen Feststoffen, unter Erhalt eines aufbereiteten Glyzerins.

2. Verfahren gemäß Anspruch 1, **dadurch gekennzeichnet, dass** das Rohglyzerin in der Stufe a) entweder bei Normaldruck auf eine Temperatur in dem Bereich von 85 °C bis 95 °C erhitzt wird oder bei verringertem Druck auf eine entsprechend verringerte Temperatur, bei der die im Rohglyzerin enthaltenen Stoffe abdestilliert werden, die bei Normaldruck eine Siedetemperatur im Bereich von 60 °C bis 80 °C aufweisen.

3. Verfahren gemäß Anspruch 1, **dadurch gekennzeichnet, dass** in der Stufe a) zunächst in einer ersten Phase a1) das Rohglyzerin unter Normaldruck auf eine Temperatur in dem Bereich von 85 °C bis 95 °C erhitzt wird und anschließend in einer zweiten Phase a2) bei verringertem Druck auf eine verringerte Temperatur, bei der die im Rohglyzerin enthaltenen Stoffe abdestilliert werden, die bei Normaldruck eine Siedetemperatur im Bereich von 60 °C bis 80 °C aufweisen.

4. Verfahren gemäß einem der Ansprüch 1 bis 3, **dadurch gekennzeichnet, dass** das Zwischenprodukt, das in der Stufe a) erzeugt wurde, in der Stufe b) entweder bei Normaldruck auf eine Temperatur in dem Bereich von 110 °C bis 140 °C erhitzt wird oder bei verringertem Druck auf eine entsprechend verringerte Temperatur, bei der die im Rohglyzerin enthaltenen Stoffe abdestilliert werden, die bei Normaldruck eine Siedetemperatur im Bereich von 80 °C bis 100 °C aufweisen.

5. Verfahren gemäß einem der Ansprüch 1 bis 3, **dadurch gekennzeichnet, dass** in der Stufe b) zunächst in einer ersten Phase b1) das Zwischenprodukt, das in der Stufe a) erzeugt wurde, unter Normaldruck auf eine Temperatur in dem Bereich von 110 °C bis 140 °C erhitzt wird und anschließend in einer zweiten Phase b2) unter verringertem Druck auf eine entsprechend verringerte Temperatur, bei der die im Rohglyzerin enthaltenen Stoffe abdestilliert werden, die bei Normaldruck eine Siedetemperatur im Bereich von 80 °C bis 100 °C aufweisen.

6. Verfahren gemäß einem der vorangehenden Ansprüche, **dadurch gekennzeichnet, dass** das Rohglyzerin vor der Stufe a) vorfiltriert wird.

7. Verfahren gemäß einem der vorangehenden Ansprüche, **dadurch gekennzeichnet, dass** die Filtration in Stufe c) unter einem absoluten Druck im Bereich von 5500 hPa bis 6500 hPa (5,5 bar absolut bis 6,5 bar absolut) erfolgt.

8. Verfahren gemäß einem der vorangehenden Ansprüche, **dadurch gekennzeichnet, dass** in Stufe c) vor dem Filtrieren Filterhilfsstoffe zugegeben werden und/oder der pH-Wert zur Optimierung der Filtration eingestellt wird.

9. Verfahren gemäß einem der vorangehenden Ansprüche, **dadurch gekennzeichnet, dass** dem nach den Stufen a) bis c) erhaltenen aufbereiteten Glyzerin ein Stoff oder Stoffgemisch mit höherem Brennwert zugesetzt wird, wobei der Stoff oder das Stoffgemisch ausgewählt ist unter Pflanzenöl, Biodiesel und Gemischen davon.

10. Verfahren gemäß einem der vorangehenden Ansprüche, **dadurch gekennzeichnet, dass** das nach den Stufen a) bis c) erhaltene aufbereitete Glyzerin in einem Verbrennungsmotor verbrannt wird, wobei mit Hilfe eines Generators elektrische Energie erzeugt wird.

11. Verfahren gemäß Anspruch 10, **dadurch gekennzeichnet, dass** durch den Verbrennungsmotor Abwärme erzeugt wird, die in den Stufen a) und/oder b) zum Erhitzen verwendet wird.

12. Verfahren gemäß einem der Ansprüche 10 bis 11, **dadurch gekennzeichnet, dass** die Abgase des Verbrennungsmotors zur Dampferzeugung für eine Stromerzeugung mittels eines Dampfgenerators verwendet werden.
